# EUROPEAN PATENT SPECIFICATION

(11) **EP 1 824 420 B1**
(45) Date of publication and mention of the grant of the patent: **15.12.2010**
(21) Application number: 04790889.2
(22) Date of filing: 27.10.2004
(51) Int. Cl.: A61F 2/30, A61L 27/56, A61L 27/58

(54) **IMPLANT FOR REPAIRING A CARTILAGE DEFECT**
IMPLANTAT ZUR REPARATUR EINES KNORPELDEFEKTS
IMPLANT POUR LA REPARATION D'UN DEFAUT DE CARTILAGE

(43) Date of publication of application: 29.08.2007
(73) Proprietor: Tetec-Tissue Engineering Technologies Aktiengesellschaft, 72772 Reutlingen (DE)
(72) Inventor: GAISSMAIER, Christoph, 72072 Tübingen (DE); FRITZ, Jürgen, 72144 Dusslingen (DE); AICHER, Wilhelm, 72119 Ammerbuch (DE)
(74) Representative: Mütschele, Thomas
(86) International application number: PCT/EP2004/012109
(87) International publication number: WO 2006/045330

(56) References cited:
- EP-A- 1 273 312
- US-B1- 6 319 712
- US-B1- 6 530 956

## Description

### Field of the invention

The invention relates to an implant for repairing a cartilage defect, and more particularly for repairing load-bearing cartilage, such as articular cartilage, comprising a first layer and a second layer, the first layer facing the synovial space and the second layer being located towards bone. The invention further relates to a method for treating a cartilage defect.

### Background of the invention

Cartilage suffers from a very limited ability for repair of joint surface damage. It typically fails to heal on its own and cartilage defects can be associated with pain, loss of function and disability.

Articular cartilage forms a layer at the surface joints. When articular cartilage is damaged due to trauma or osteochondrosis, normally defined surface defects exist while the surrounding of the cartilage is intact.

Conventional treatment options include, for example, debridement, which involves the removal of synovial membrane, osteophytes, loose articular debris, and diseased cartilage. Using this technique symptomatic relief can be achieved only. Further treatments include subchondral drilling, microfracture and abrasion arthroplasty; by using these techniques, the subchondral bone is perforated and penetrated to induce bleeding and plot formation. In doing so, it is attempted to restore the articular surface by inducing growth of fibrocartilage into the damaged cartilage area.

Nevertheless, the capability of fibrocartilage to withstand shock or shearing forces is low compared to hyaline cartilage. Further, fibrocartilage is degenerating over the time, and resulting in the return of clinical syndromes.

Other techniques, such as transplantation of periosteum or perichondrium have not been proven to be suitable, and using allogenic cartilage-bone-transplants should be avoided due to the high risk of infection or rejection of the transplants.

Yet another approach is the method of autologous osteochondral transfer (mosaicplasty), whereby cylinders of normal cartilage and bone from "non-weight-bearing" areas of, for example, an affected knee, are removed and placed into cartilage defects during a single surgical procedure. These "autografts" result in the formation of a patchwork or mosaic.

However, this method is rather limited due to fact that healthy tissue is not available indefinitely and that very often the congruence of the cartilage surfaces is not satisfying.

Another treatment is autologous chondrocytes transplantation (ACT). By using this method it is attempted to regenerate hyaline-like cartilage and thereby restore function. When performing this method, a region of healthy articular cartilage is identified and biopsied by arthroscopy. Out of the biopsied cartilage, chondrocytes are separated and subsequently cultivated in culture medium. After two to three weeks an arthrotomy is performed and the chondral lesion is excised up to the healthy surrounding cartilage. A periosteal flap is removed from, for example, the proximal medial tibia, and is sutured over the defect to the surrounding rim of normal cartilage to form a lid. The cultured chondrocytes are then injected beneath the periosteal flap. As a result, cartilage is evolving, which is - in view of histological and biomechanical features - similar to articular cartilage after a while. The advantage of this procedure is that even large defect areas can be restored effectively.

In spite of the achieved promising results, the conventional ACT method reveals disadvantages and obstacles. The arthrotomy - while often being enduring - is accompanied by postoperative complaints. Further, often the periosteal flap cannot be sutured over the defect, especially when the containment is missing.

In addition, there is a risk that the defect has not been replaced properly or that the transplant is even collapsing, due to the fact that, for example, the cultured chondrocytes are of minor quality or that the periosteal flap is shearing prematurely.

In EP 0 934 750 A2, a biohybrid articular surface replacement device is disclosed comprising a three dimensional porous carrier suitable for culturing cartilage cells and bone integration means provided on the side of the porous carrier intended for engagement with the bone. Further, the device disclosed in EP 0 934 750 A2 can comprise a cover film intended for covering the top side of the carrier remote from the bone integration means. The disclosed cover film serves as a barrier to prevent synovial fluids to permeate the replacement device.

The replacement device of EP 0 934 750 A2 has the disadvantage that by providing the device with a cover film as disclosed the device is at risk not getting supplied with nutrients and/or fluids essential for maintaining functional features of the replacement device.

Currently, there are no satisfying treatments for full-thickness lesions of articular cartilage, since the available methods are generally regarded as being not effective, excessively expensive or have other problems associated therewith.

In US 6,530,956 B1, a resorbable scaffold is disclosed that promotes cartilage regeneration. The scaffold uses two distinct matrix materials, the first one being relatively stiff and being designed to provide an outer rim and one or more internal runners, rim and runners creating a cluster of internal cellrank compartments which are filled with the second material, a more open and porous matrix. The scaffold can optionally also comprise an articulating outer membrane resting on the upper edges of the runners and rim. The scaffold is supporting the membrane which is mimicking a healthy cartilage surface.

In US 6,319,712 B1, an implant device is disclosed comprising a porous carrier and a bone integration aid being provided on a side of the porous carrier intended for engagement with bone.

In EP 1 273 312 A2, an implant for cartilage tissue regeneration is disclosed comprising a mesh or porous sponge of a synthetic polymer and a porous sponge formed on or in the mesh or porous sponge.

### Summary of the invention

In view of the above it is an object of the present invention to overcome the mentioned disadvantages and to provide for an effective and reliable treatment for repairing cartilage defects.

This object is, according to the invention, achieved by providing an implant for repairing a cartilage defect consisting of a first layer and a second layer, wherein said first layer is a protective membrane-like barrier structure and said second layer has a sponge-like structure comprising directional and/or interconnected pores and wherein said first layer is adapted to face the synovial space and said second layer is adapted to be located towards bone.

The object underlying the invention is completely achieved in this fashion.

Specifically, with an implant of this kind, a homogenous three-dimensional distribution of cells growing into the pores of the sponge-like structure can be achieved. On the other hand, the membrane-like structure provides a barrier for non-specific connective tissue cells, which would otherwise infiltrate the implant, for example from the synovial space.

The inventors of the present invention have shown that the implant according to the invention was superior to commercially available implants of different manufacturers. Most of the conventional implants failed to provide for a homogenous and three-dimensional distribution of cells previously cultured in monolayers and then applied into the implants. Rather, in the conventional implants, cells were concentrating at the surface area of the layer that is supposed to carry the cells due to the filter effect caused by the conventional layer's structure. With the porous structure of the sponge-like "carrier"-layer of the present invention's implant this disadvantage can be avoided.

Further, the sponge-like structure renders the implant flexible or compressible in that way, that if the implant is set under strain, fluids are given off the sponge-like structure and are taken up again after decompression. As a result, after transplantation cells can be supplied with nutrients *in vivo* in a natural way.

With an implant according to the invention the disadvantages of conventional ACT can be overcome: the implant can be transplanted under minimal invasive conditions and without putting too much strain on soft tissue, joint capsule and ligaments. Further, surgical time needed for the treatment is significantly reduced. In addition, when using an implant according to the invention periosteal flaps don't have to be sutured any more over the defect for cell fixation, and as a result secondary hypertrophies can be avoided.

Further, due to the minimal surgical access and the therefore shortened healing process patients can return to normal activity earlier than it is the case for patients treated with conventional ACT.

Reference herein to "directional and/or interconnected pores" of the sponge-like structure is to pores which are orientated, e.g. in the manner of a hollow fibre structure, and/or which are mutually connected. In that way the pores - like fibres - run roughly parallel to each other while having a regular or consistent pore size; at the same time the pores can be mutually connected. Due to the interconnection between the pores, cells migrating into the implant are distributed not only within the directional pores but can rather also attach to the interconnection zones between the pores thereby providing a homogenous three-dimensional distribution of the cells in the implant.

It is preferred to use the implant according to the invention for replenishment of cartilage defects in knee, invertebral disks or in other joints of the human body.

Suitably, the first layer and second layer of the implant of the present invention each comprise biocompatible materials.

It is further preferred, when said first layer comprises a material having a resorption time exceeding the resorption time of said second layer.

Reference herein to "resorption time" is to the time the material needs to be broken down due to biochemical/biological reactions *in vivo.* As a result the implants don't have to be removed by means of an additional surgery.

The advantage here is, that the second layer, carrying the cells, is protected during its resorption by the first layer's membrane-like structure, so that undesirable tissue fluids or cells, for example from the synovial space, cannot infiltrate the implant, which would otherwise lead to a weakened implant. The second layer remains protected till it is replenished with matrix of cartilage produced by the transplanted cartilage cells.

Additionally, the first layer represents a diffusion barrier for high molecular weight substances, thereby retaining the components of the matrix of cartilage produced by the transplanted cells and promoting efficient defect replenishment. Over the healing process the implant is resorbed completely and without any toxic metabolites.

It is preferred, when said first layer comprises a material which is selected from the group consisting of collagen, bioresorbable polymers, pericardium, composites, glycosaminoglycanes, natural tissue sources like elastin, and mixtures of two or more of these materials.

Further, blood born components such as fibrin can be included in the first layer.

Advantageously, with the first layer - having a resorption time exceeding the resorption time of the second layer - a stable scaffolding is provided, which is suitable to fix the implant via pins or sutures to the defect site. As a result, the patient's site into which the implant has been introduced can be strained right after the surgical procedure.

In a preferred form of the implant according to the invention the second layer comprises a hydrophilic material, in particular a material which is selected from the group consisting of collagen, hyaluronic acid, alginate, chitosan, gelatine, processed materials, composites, blood born components such as fibrin, and mixtures of two or more of these materials.

The mentioned materials have proven to be biocompatible, bioresorbable in definite time, and suitable for implantation purposes and are known in the art. Collagen is a major protein constituent of connective tissue in vertebrate as well as in invertebrate animals. According to the present invention collagen from any source is suitable, including commercially available collagen.

With the sponge-like second layer comprising a hydrophilic material the uptake of substances and/or fluids is facilitated, since after compressing the structure - e.g. via strain - and subsequently releasing it, inflow of substances and/or cells into the sponge-like structure is generated.

It is further preferred when the second layer further comprises substances which are selected from the group consisting of antiangiogenetic agents, morphogenic agents, mitogenic and antiinflammatoric agents, or mixtures of two or more of these substances.

With the mentioned substances integration of the implant in the surrounding cartilage can be facilitated.

In addition, physiological components of bone may also be included in the implant, in particular in the second layer of the implant. These components include, but are not limited to, calcium phosphates, calcium sulphates, calcium fluorides, calcium oxides, hydroxyl apatites, or mixtures of two or more of these components. The calcium phosphate compounds which can be used include, for example, tricalcium phosphate and tetracalcium triphosphate, combined with calcium hydroxide. The purpose of the addition of these compounds is to stabilize the phenotype of the cells once introduced in the implant.

It is further preferred if the pore structure of the second layer's sponge-like structure comprises pores of the size of about 50 µm to about 250 µm, and more preferably of about 130 µm to 200 µm.

The pore size and structure depend on and may be adjusted to the cells preferably to be taken up or to be seeded into the implant. With the directional and/or interconnected pore structure the implant can be modulated in consideration of the respective environment the implant is transplanted into, i.e. the pore size can be adapted to the size of the cells with which the implant is to be seeded with or which are desirable to migrate into the implant.

A suitable implant of the present invention comprises a sponge-like structure with a pore structure which is suitable to be seeded with cells, preferably with chondrocytes, chondroprogenitor cells, stem-cells, cells from periosteum tissue, and cells from perichondrium tissue or mixtures of two or more of these cell types.

With an implant according to the present invention it is on one hand possible to seed the implant with the mentioned cells (or a mixture of two or more different cell types) prior to its implantation into the cartilage defect.

On the other hand, the implant can be placed directly into the defect without having cells seeded onto it. After transplantation of the latter embodiment of the implant of the present invention and after putting it under normal strain the implant is - due to its sponge-like structure - compressed and relaxed repeatedly whereby substances such as cells, fluids, nutrients, etc., which are present in the defect site, are taken up. The pore structure of the implant's second layer provides for effective absorption of the cells, so that the cells can grow and/or migrate into the pores due to the hollow fibre structure of said pores. This is due to the fact that - as mentioned above - the implant - being compressible - is pressed and relaxed *in vivo* in the mode of squeezing a sponge.

The implant according to the invention can be fixed to the defect site by, e.g., suturing, using pins, or the like. On the other hand, it is possible to insert the implant without fixation to the defect site. It can be sufficient to just place the implant on the defect site in order to provide for fixation via adsorption.

Yet another aspect of the present invention provides for an implant, in which the cells are taken from the patient into whom the implant is introduced, that is the cells are autologous.

Another object of the present invention is to provide for an implant to be seeded with cells that are taken from a source that is heterologous or to the patient into whom the implant is to be introduced.

A further object of the invention provides for an implant to be seeded with cells that are taken from a source that is xenogenic to the patient into whom the implant is to be introduced.

These embodiments have the advantage that - in case the patient into whom the implant is to be introduced cannot provide for suitable donor cells himself/herself - suitable cells of other sources may be utilized.

It is understood that with respect of this embodiment of the present invention cells have to be selected which are compatible to the tissue and cell structure of the patient, into whom the implant is to be introduced.

The implant of the present invention may further have a first layer having a depth of about 0.01 mm to about 0.5 mm.

Further, the implant of the present invention may comprise a second layer having a depth of about 0.3 mm to 3.5 mm.

It is further preferred when the implant's first and second layer are fixed to one another. Preferably, fixation can be achieved, for example, by using adhesives, by suturing, or any other fixation mechanism known in the art. Fixation can further be accomplished by applying the second layer on the first layer followed by a lyophilising or precipitating step.

The directional and/or interconnected pore structure of the second layer is desirable since, as mentioned above, the structure can be optimally matched to the given tissue. Matching the structure and size of the pores improves the migratory behavior of the surrounding tissue.

Another object of the invention relates to a method for producing a cartilage implant according to the invention.

As mentioned above, the implant according to the invention can be seeded with cells prior to the implantation process. For this purpose, chondrocytes can be taken from a patient, for example the patient, into whom the implant is to be introduced afterwards, followed by seeding those chondrocytes in a cell culture assay, cultivating and proliferating them.

In parallel, an implant according to the invention is provided, onto which the cells can then be supplied. This is performed in such a way that the cells are seeded on the sponge-like structure of the implant with the first layer facing the supporting base. Since the second structure is bordered by the membrane-like first structure, the cells applied on the sponge-like structure cannot fall through the pores onto the supporting base. Further, the pore size of the sponge-like structure is preferably adapted to the size of the cells, so that the cells can adhere to the pores and grow in the pores along the hollow fibre structure of the pores. Due to the adapted and orientated pores the cells can be evenly distributed over the sponge-like second layer.

It is further described a method for treating a cartilage defect comprising the steps of
a) providing an implant in a vessel, wherein the implant comprises a first and at least a second layer, wherein said first layer comprises a membrane-like structure and said second layer comprises a sponge-like structure having directional and/or interconnected pores of a size of about 50 µm to about 250 µm and wherein said first layer is facing the synovial space and said second layer is located towards bone/cartilage, and wherein the first layer comprises a material having a resorption time exceeding the resorption time of the second layer;
b) cutting the defect cartilage of a patient, preferably with a punching device, and subsequent curettage to obtain a defect contact surface;
c) cutting the implant to the size as the defect contact surface obtained in step b); and
d) introducing the implant onto the defect contact surface.

When using this technique it is preferred to perform a microfracture prior to transplantation, i.e. the subchondral bone, which is lying under the cartilage, is penetrated so that blood and cells are exuding from the bone marrow. The blood stimulates the production of cartilage and the cells, i.e. progenitor cells, adhere to the bone and subsequently to the implant, into which they can migrate. Via corresponding differentiation of these progenitor cells generation of fibrocartilage is facilitated.

When applying the mentioned technique, the implant is carefully placed onto the defect contact surface, which was prior treated with microfracture. After putting normal strain on the transplant site the implant is pressed and released, whereby a sponge effect is produced, i.e. cells being present on the defect contact surface are being taken up by the sponge-like structure of the second layer into the pores. However, it has to be stated, that microfracture can only be performed in non-inflammatory indications and in cartilage defects being smaller than 2 cm².

Still it is described a method that comprises a further step a') comprising the step of seeding and cultivating cells on the implant to obtain a cell-seeded implant.

Using this method, the implant is seeded with cells before the cell-seeded implant is then introduced into the defect cartilage site. When using this method, microfracture of the subchondral bone is not necessary, since the implant already contains cells prior to transplantation.

Cells being suitable for this method may be cells which are selected from the group consisting of chondrocytes, chondroprogenitor cells, bone-precursor cells, stem-cells, cells from periosteum tissue, cells from perichondrium tissue, and progenitor cells from blood. Further, two or more different cell types may be used within the method. The cells, as mentioned above, may be taken from the patient into whom the implant is to be introduced or from a source that is heterologous to the patient into whom the implant is to be introduced.

With the above mentioned method it may not be necessary to affix the implant to the defect with fixation means due to the adhesive features of the implant.

In yet another example the above-mentioned method comprises the step of
e) affixing the implant to the defect cartilage by fixation means which are selected from the group consisting of stitches, pins and tissue adhesives.

In a preferred embodiment the fixation means comprise a biocompatible material. Depending on the defect's condition and localisation two or more different kinds of the fixation means may be combined with each other.

The mentioned method may be applied for the replenishment of cartilage defects in knee, invertebral disks or in other joints of the human body.

Further advantages can be taken from the following description and figures.

It goes without saying that the features, mentioned above, and those which are still to be explained below, can be used not only in the combination which is in each case specified but also in other combinations, or on their own, without departing from the scope of the present invention.

### Brief description of the drawings

- Fig. 1: shows an implant according to the invention consisting of a first membrane-like structure (A) and a second sponge-like structure (A', A"); in (B) the distribu- tion of fluorescence labelled cells in an implant ac- cording to the invention in shown;
- Fig. 2: shows expression of IL-1 (A) and collagen type II (B) of chondrocytes cultured in an implant according to the invention ("TETEC") and in commercially available implants ("T1", "T2");
- Fig. 3: shows the protection of the second layer during its resorption till the defect's replenishment (A) (sa- franine o staining) and (B) (hematoxilin/eosin stain- ing); the second layer is marked with arrows 2 and seeded with cells, the first layer is marked with ar- rows 1 and functions as protective layer for cells and matrix underneath; (C) (safranine o staining) shows the reestablishment of hyaline cartilage using an implant according to the invention.

### Detailed Description of Preferred Embodiments

### Example 1

### Preparation of an implant according to the invention

An implant according to the invention can be prepared - for example - by a method disclosed in EP 1 275 405. By using this method a sponge-like layer or protein matrix can be anchored in a membrane-like layer. Briefly, a membrane-like layer was provided comprising collagen - other suitable materials are, e.g., bioresorbable polymers such as polylactide or polyglycolic acid, collagen, pericardium, composites, glycosaminoglycanes, natural tissue sources like elastin, and mixtures of two or more of these materials. The sponge-like layer was applied thereon in form of a suspension. Alternatively it can be supplied as a dispersion or paste. The suspension was comprising collagen - other materials can be used, e.g. hyaluronic acid, alginate, chitosan, gelatine, processed materials, composites, blood born components such as fibrin, and mixtures of two or more of these materials - and was introduced into the membrane-like structure by means of pressure vacuum. Alternatively, the suspension, dispersion or paste can be introduced by centrifugation. Subsequently, the sponge-like structure of the second layer was formed by unilaterally cooling the membrane-like structure with the suspension applied thereon. The cooling process was performed by gradually lowering the temperature from room temperature to - 50 °C thereby generating directional and/or interconnected pores.

The implant produced in that way was seeded with chondrocytes; alternatively it can be seeded with other cells, e.g. with chondroprogenitor cells, stem-cells, cells from periosteum tissue, and cells from perichondrium tissue or mixtures of two or more of these cell types, before introducing it into the defect site. On the other hand, the implant can be placed directly into the defect without having cells seeded onto it. In the latter case, after putting it under normal strain the implant is compressed and relaxed repeatedly whereby substances such as cells, fluids, nutrients, etc., which are present in the defect site, are taken up. Due to the hollow fibre structure of the sponge-like structure's pores cells can grow and/or migrate into the pores resulting in a homogenous three-dimensional distribution of the cells.

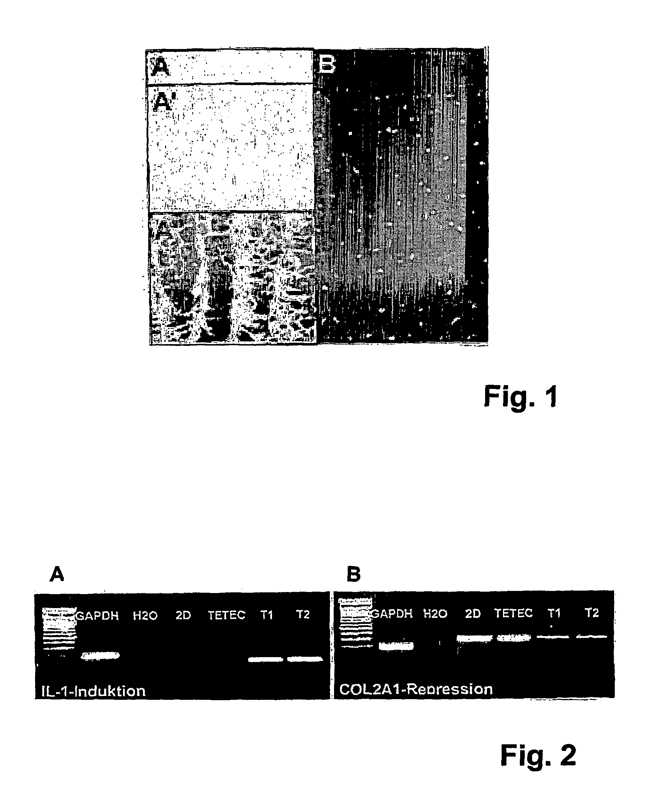

In Fig. 1 the layers of the implant prepared accordingly are shown. The implant comprises a membrane-like first layer (A) serving as a cover for the underlying sponge-like second layer (A') that comprises pores orientated directional with respect to the surface in a column-like fashion. First layer and second layer are tightly connected. The pores are interconnected and comprise a directional pore size, whereby a three dimensional distribution of cells can be achieved. The upper picture of the porous layer A' was taken using transmitted light microscopy, the lower picture of the porous layer (A'') was taken using scanning electron microscopy.

In Fig. 1, B shows the distribution of fluorescence labelled chondrocytes within the sponge-like second layer of the implant. When preparing the implant the base of the second layer (for example collagen) can comprise additional physiological components of the hyaline cartilage to enhance stable regeneration.

As can be seen in pictures A' and A" of Fig. 1, the pores of the sponge-like structure are directional and interconnected. In that way a homogenous distribution of the cells growing into or migrating into the implant can be achieved which is shown in picture B of Fig. 1.

### Example 2

With respect to the biocompatibility of the implants, induction of Interleukin IL-1 expression and reduction of collagen type II expression of chondrocytes seeded into different implants was assessed *in vitro.*

The results of these tests are shown in Fig. 2. In fig. 2A ("IL-1 Induktion") it is shown that IL-1 induction in chondrocytes seeded on an implant according to the invention ("TETEC") is lower than IL-1 expression in chondrocytes seeded on commercially available implants ("T1" and "T2). When IL-1 expression was increased hypertrophy and degeneration of chondrocytes seeded into the implants could be observed *in vitro*. A marker and controls "GAPDH" and "H₂O" are displayed in lanes 1 to 3 respectively.

Further, expression of collagen type II, which is an essential structural protein in cartilage, was remarkably reduced in chondrocytes seeded into commercially available implants ("T1" and "T2") in comparison to chondrocytes seeded into implants according to the invention ("TETEC"). These results are shown in Fig. 2B ("COL2A1-Expression"). In Fig. 2B, a marker and controls "GAPDH" and "H₂O" are displayed in lanes 1 to 3 respectively.

### Example 3

The implant prepared as mentioned above (see Example 1) was tested in animal studies. Experiments were conducted in SCID ("severe combined immunodeficiency") mice, into which human cells can be transplanted without rejection of these cells, since in the mentioned mice the enzyme adenosine deaminase is deficient and - as a result - T or B cells are not being developed.

It was previously shown in SCID mice that human articular chondrocytes do only produce solid hyaline cartilage when the transplanted cells express certain marker genes, the fact of which has to be proofed in molecular/biological quality assays. Chondrocytes not expressing collagen type II, BMP-2 (bone morphogenetic protein 2) and FGFR-3 (fibroblast growth factor receptor 3) any more are not able to regenerate high quality cartilage.

In test group A, human articular chondrocytes expressing relevant marker genes were seeded on an implant according to the invention (5 x 10⁵ cells/cm² carrier layer, i.e. second layer). The implant was subsequently transplanted into SCID mice. After incubation in SCID mice, high quality hyaline cartilage was consistently formed, which could be proofed by hematoxilin/eosin staining and safranine o staining. When implanting an implant without the membrane-like first layer, immigration of unspecific connective tissue cells into the sponge-like structure was observed leading to a softening of the implant.

In control group B, transplantation of human articular chondrocytes not expressing the relevant marker genes any more (5 x 10⁵ cells/cm² carrier (layer)) resulted in a clearly inferior and inhomogeneous regeneration, which was shown by hematoxilin/eosin staining and safranine o staining as well.

### Example 4

After transplantation, the healing process was checked for resorption of the implant and replenishment of the cartilage defect in animal model (mouse).

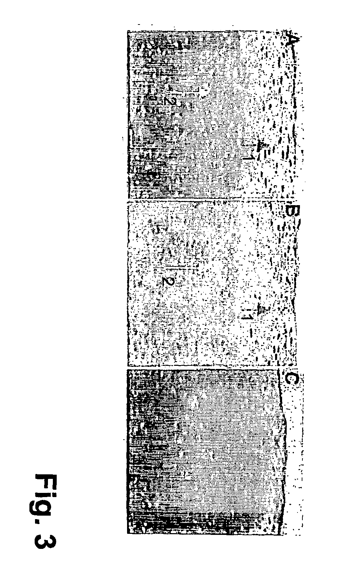

To control the healing process, dissection of the implant after a retention time of about 8 to 12 weeks in the defect cartilage and section staining was performed. The results are shown in Fig. 3. As can be seen in Fig. 3A (safranine o staining) and Fig. 3B (hematoxilin/eosin staining), the membrane-like first layer (indicated by arrows 1) protects the sponge-like second layer underneath and functions as a barrier for cells, thereby ensuring that the underlying sponge-like second layer is kept in a stable condition (indicated by arrows 2) required for replenishment of the cartilage.

After 8 to 12 weeks the transplantation site was checked for regeneration. Complete resorption of all portions of the implant could be observed, which is shown in Fig. 3C (safranine o staining). The defect site was completely recovered and replenished with hyaline cartilage.

## Claims

1. An implant for repairing a cartilage defect consisting of a first layer and a second layer, wherein said first layer is a protective membrane-like barrier structure, which provides a barrier for non-specific connective tissue cells, and said second layer has a sponge-like structure comprising directional and/or interconnected pores and wherein said first layer is adapted to face the synovial space and said second layer is adapted to be located towards bone.

2. The implant according to claim 1, wherein said first layer and said second layer each comprises biocompatible materials.

3. The implant according to claim 1 or 2, wherein said first layer comprises a material having a resorption time exceeding the resorption time of said second layer.

4. The implant according to any of claims 1 to 3, wherein said first layer comprises a material which is selected from the group consisting of collagen, bioresorbable polymers, pericardium, composites and glycosaminoglycanes, or mixtures of two or more of these materials.

5. The implant according to any of claims 1 to 4, wherein said second layer comprises a hydrophilic material.

6. The implant according to claim 5, wherein said second layer comprises a material which is selected from the group consisting of collagen, hyaluronic acid, alginate, chitosan, gelatine, blood born components, processed materials and composites, or mixtures of two or more of these materials.

7. The implant according to any of claims 1 to 6, wherein said second layer further comprises substances which are selected from the group consisting of antiangiogenesis, morphogenic, mitogenic, and antiinflammatoric agents, and mixtures of two or more of these substances.

8. The implant according to any of claims 1 to 7, wherein said pore structure comprises pores of a size of about 50 µm to about 250 µm.

9. The implant according to claim 8, wherein said pore structure comprises pores of a size of about 130 µm to about 200 µm.

10. The implant according to any of claims 1 to 9, wherein said sponge-like structure is suitable to be seeded with cells.

11. The implant according to claim 10, wherein said cells are selected from the group consisting of chondrocytes, chondroprogenitor cells, bone-precursor cells, stem cells, cells from periosteum tissue, and cells from perichondrium tissue, or mixtures of two or more of these cell types.

12. The implant according to claims 10 or 11, wherein said cells are taken from the patient into whom the implant is to be introduced.

13. The implant according to claim 10 or 11, in which said cells are taken from a source which is heterologous to the patient into whom the implant is to be introduced.

14. The implant according to claim 10 or 11, in which said cells are taken from a source which is xenogenic to the patient into whom the implant is to be introduced.

15. The implant according to any of claims 1 to 14, in which said first layer is having a depth of about 0.01 mm to about 0.5 mm.

16. The implant according to any of claims 1 to 15, in which said second layer is having a depth of about 0.3 mm to about 3 mm.

17. The implant according to any of claims 1 to 16, wherein said first and said second layer are fixed to one another.

## Patentansprüche

1. Implantat zur Reparatur eines Knorpeldefekts, gebildet aus einer ersten Schicht und einer zweiten Schicht, wobei die erste Schicht eine schützende membranartige Barrierestruktur ist, die eine Barriere für unspezifische Bindegewebszellen bildet, und die zweite Schicht eine schwammartige Struktur aufweist, die gerichtete und/oder zusammenhängende Poren umfasst, und wobei die erste Schicht dazu ausgebildet ist, dass sie dem Synovialraum zugewandt ist, und die zweite Schicht dazu ausgebildet ist, dass sie zum Knochen gelegen ist.

2. Implantat nach Anspruch 1, wobei die erste Schicht und die zweite Schicht jeweils bioverträgliche Materialien umfassen.

3. Implantat nach Anspruch 1 oder 2, wobei die erste Schicht ein Material umfasst, das eine höhere Resorptionszeit aufweist als die Resorptionszeit der zweiten Schicht.

4. Implantat nach einem der Ansprüche 1 bis 3, wobei die erste Schicht ein Material umfasst, das ausgewählt ist aus der Gruppe bestehend aus Kollagen, bioresorbierbaren Polymeren, Perikardium, Verbundstoffen und Glycosaminoglycanen oder Mischungen von zwei oder mehreren dieser Materialien.

5. Implantat nach einem der Ansprüche 1 bis 4, wobei die zweite Schicht ein hydrophiles Material umfasst.

6. Implantat nach Anspruch 5, wobei die zweite Schicht ein Material umfasst, das ausgewählt ist aus der Gruppe bestehend aus Kollagen, Hyaluronsäure, Alginat, Chitosan, Gelatine, Blutkomponenten, aufbereiteten Materialien und Verbundstoffen oder Mischungen von zwei oder mehreren dieser Materialien.

7. Implantat nach einem der Ansprüche 1 bis 6, wobei die zweite Schicht weiter Substanzen umfasst, die ausgewählt sind aus der Gruppe bestehend aus Antiangiogenesemitteln, Morphogenen, Mitogenen und entzündungshemmenden Mitteln und Mischungen von zwei oder mehreren dieser Substanzen.

8. Implantat nach einem der Ansprüche 1 bis 7, wobei die Porenstruktur Poren einer Größe von ungefähr 50 µm bis ungefähr 250 µm umfasst.

9. Implantat nach Anspruch 8, wobei die Porenstruktur Poren einer Größe von ungefähr 130 µm bis ungefähr 200 µm umfasst.

10. Implantat nach einem der Ansprüche 1 bis 9, wobei die schwammartige Struktur zur Besiedlung mit Zellen geeignet ist.

11. Implantat nach Anspruch 10, wobei die Zellen ausgewählt sind aus der Gruppe bestehend aus Chondrozyten, Chondroprogenitorzellen, Knochenvorstufenzellen, Stammzellen, Zellen aus Periosteumgewebe und Zellen aus Perichondriumgewebe oder Mischungen von zwei oder mehreren dieser Zelltypen.

12. Implantat nach Anspruch 10 oder 11, wobei die Zellen dem Patienten entnommen sind, dem das Implantat eingesetzt werden soll.

13. Implantat nach Anspruch 10 oder 11, wobei die Zellen von einer Quelle gewonnen sind, die zum Patienten, dem das Implantat eingesetzt werden soll, heterolog ist.

14. Implantat nach Anspruch 10 oder 11, wobei die Zellen von einer Quelle gewonnen sind, die zum Patienten, dem das Implantat eingesetzt werden soll, xenogen ist.

15. Implantat nach einem der Ansprüche 1 bis 14, wobei die erste Schicht eine Tiefe von ungefähr 0,01 mm bis ungefähr 0,5 mm aufweist.

16. Implantat nach einem der Ansprüche 1 bis 15, wobei die zweite Schicht eine Tiefe von ungefähr 0,3 mm bis ungefähr 3 mm aufweist.

17. Implantat nach einem der Ansprüche 1 bis 16, wobei die erste und die zweite Schicht miteinander verbunden sind.

## Revendications

1. Implant destiné à réparer un défaut de cartilage constitué d'une première couche et d'une seconde couche, dans lequel ladite première couche est une structure barrière de type membrane protectrice, qui constitue une barrière pour des cellules du tissu conjonctif non spécifiques, et ladite seconde couche a une structure spongieuse comprenant des pores directionnels et/ou communicants et dans lequel ladite première couche est conçue pour faire face à l'espace synovial et ladite seconde couche est conçue pour se trouver en regard de l'os.

2. Implant selon la revendication 1, dans lequel ladite première couche et ladite seconde couche comprennent chacune des matières biocompatibles.

3. Implant selon la revendication 1 ou 2, dans lequel ladite première couche comprend une matière ayant un temps de résorption supérieur au temps de résorption de ladite seconde couche.

4. Implant selon l'une quelconque des revendications 1 à 3, dans lequel ladite première couche comprend une matière qui est choisie dans le groupe constitué par le collagène, les polymères biorésorbables, le péricarde, les composites et les glycosaminoglycanes, ou les mélanges de deux ou plus de deux de ces matières.

5. Implant selon l'une quelconque des revendications 1 à 4, dans lequel ladite seconde couche comprend une matière hydrophile.

6. Implant selon la revendication 5, dans lequel ladite seconde couche comprend une matière qui est choisie dans le groupe constitué par le collagène, l'acide hyaluronique, les alginates, le chitosane, la gélatine, les composants issus du sang, les matières transformées et les composites, ou les mélanges de deux ou plus de deux de ces matières.

7. Implant selon l'une quelconque des revendications 1 à 6, dans lequel ladite seconde couche comprend en outre des substances qui sont choisies dans le groupe constitué par les agents antiangiogéniques, morphogénétiques, mitogénétiques et anti-inflammatoires, et les mélanges de deux ou plus de deux de ces substances.

8. Implant selon l'une quelconque des revendications 1 à 7, dans lequel ladite structure poreuse comprend des pores ayant une taille d'environ 50 µm à environ 250 µm.

9. Implant selon la revendication 8, dans lequel ladite structure poreuse comprend des pores ayant une taille d'environ 130 µm à environ 200 µm.

10. Implant selon l'une quelconque des revendications 1 à 9, dans lequel ladite structure spongieuse est appropriée pour être ensemencée de cellules.

11. Implant selon la revendication 10, dans lequel lesdites cellules sont choisies dans le groupe constitué par les chondrocytes, les cellules chondroprogénitrices, les cellules précurseurs osseux, les cellules souches, les cellules provenant de tissu de périoste et les cellules provenant de tissu de périchondre, ou les mélanges de deux ou plus de deux de ces types de cellules.

12. Implant selon les revendications 10 ou 11, dans lequel lesdites cellules sont prélevées chez le patient chez qui l'implant doit être introduit.

13. Implant selon la revendication 10 ou 11, dans lequel lesdites cellules sont prélevées à partir d'une source qui est hétérologue par rapport au patient chez qui l'implant doit être introduit.

14. Implant selon la revendication 10 ou 11, dans lequel lesdites cellules sont prélevées à partir d'une source qui est xénogénique par rapport au patient chez qui l'implant doit être introduit.

15. Implant selon l'une quelconque des revendications 1 à 14, dans lequel ladite première couche a une épaisseur d'environ 0,01 mm à environ 0,5 mm.

16. Implant selon l'une quelconque des revendications 1 à 15, dans lequel ladite seconde couche a une épaisseur d'environ 0,3 mm à environ 3 mm.

17. Implant selon l'une quelconque des revendications 1 à 16, dans lequel ladite première couche et ladite seconde couche sont fixées l'une à l'autre.
